Europäisches Patentamt

European Patent Office

Office européen des brevets

(19)

(11) **EP 0 850 213 B1**

(12) **EUROPÄISCHE PATENTSCHRIFT**

(45) Veröffentlichungstag und Bekanntmachung des Hinweises auf die Patenterteilung:
**27.09.2000   Patentblatt 2000/39**

(51) Int Cl.⁷: **C07C 67/54**, C07C 69/716

(21) Anmeldenummer: **96928377.9**

(86) Internationale Anmeldenummer:
**PCT/EP96/03290**

(22) Anmeldetag: **26.07.1996**

(87) Internationale Veröffentlichungsnummer:
**WO 97/06126 (20.02.1997 Gazette 1997/09)**

(54) **VERFAHREN ZUR KONTINUIERLICHEN REINDARSTELLUNG VON 5-FORMYLVALERIANSÄUREESTERN**

PROCESS FOR THE CONTINUOUS PREPARATION OF PURE 5-FORMYL VALERIC ACID ESTERS

PROCEDE DE PREPARATION EN CONTINU D'ESTERS D'ACIDE FORMYLVALERIQUE-5 PURS

(84) Benannte Vertragsstaaten:
**BE DE ES FR GB IT NL**

(30) Priorität: **09.08.1995  DE 19529239**

(43) Veröffentlichungstag der Anmeldung:
**01.07.1998   Patentblatt 1998/27**

(73) Patentinhaber: **BASF AKTIENGESELLSCHAFT
67056 Ludwigshafen (DE)**

(72) Erfinder:
• **ACHHAMMER, Günther
  D-68309 Mannheim (DE)**
• **RÖPER, Michael
  D-67157 Wachenheim (DE)**

(56) Entgegenhaltungen:
**EP-A- 0 295 551**

## Beschreibung

Die vorliegende Erfindung betrifft ein Verfahren zur Herstellung von 5-Formylvaleriansäureester in einer Ausbeute von nicht kleiner als 90 % durch Destillation eines Formylvaleriansäureestergemisches aus 5-Formylvaleriansäureester und entweder 3- oder 4- Formylvaleriansäureester oder einem Gemisch aus 3- und 4-Formylvaleriansäureestern, wobei die Esterreste der jeweiligen Formylvaleriansäureester identisch sind.

5-Formylvaleriansäureester ("5-FVSE") sind wichtige Zwischenprodukte bei der Herstellung von Adipinsäure und Caprolactam, mithin für die Herstellung von Polyamid-6,6 und Polycaprolactam. Man erhält 5-FVSE in der Regel durch Hydroformylierung von 4-Pentensäureestern im Gemisch mit den isomeren 3- und 4-Formylvaleriansäureestern. 4-Pentensäureester wiederum sind im allgemeinen durch Isomerisierung von 3-Pentensäureestern zugänglich, die ihrerseits durch Carbonylierung von Butadien zugänglich sind. Für die Herstellung von Adipinsäure und Caprolactam aus 5-FVSE ist entscheidend, daß das 5-FVSE in hoher Reinheit vorliegt. Insbesondere stören Anteile an den entsprechenden isomeren Verbindungen, besonders 4-Formylvaleriansäureester (4-FVSE), bei der Herstellung von Caprolactam über die 6-Aminocapronsäureester, da nach bisherigen Beobachtungen die Kennzahlen für das Caprolactam wie die UV-Kennzahl sowie die Fadenqualität, beispielsweise ausgedrückt durch die Fadenlänge, von Polycaprolactam verschlechtert werden, wenn die 5-FVSE keine ausreichende Reinheit aufweisen.

Die Differenzen der Siedepunkte der isomeren Formylvaleriansäureester unter Normaldruck liegen für die $C_1$-$C_2$-Alkylester im Bereich von 2 bis 5°C. So beträgt die Siedepunktsdifferenz beispielsweise bei den entsprechenden Formylvaleriansäuremethylestern (5-FVSE: 221,2°C; 4-FVSE: 223,6°C) nur 2,4°C. Somit kommt eine destillative Abtrennung des 5-FVSE von seinen isomeren 3- und 4-Formylvaleriansäureestern unter Normaldruck großtechnisch nicht in Betracht.

Hat die Siedepunktsdifferenz von zwei vorgegebenen homologen oder isomeren Verbindungen bei einem Druck p1 den Wert sd1, und sei die Siedepunktsdifferenz der gleichen zu betrachtenden Verbindungen bei einem Druck p2, wobei p2 < p1 sei, sd2, dann gilt sd2 < sd1 (siehe R.H.Perry, D.Green, Perry's Chemical Engineers Handbook, 6th Ed., 1984, Chapter 13, p. 17, Fig. 13-14). Somit kommt eine Destillation unter vermindertem Druck ebenfalls nicht in Betracht, da die Siedepunktsdifferenzen mit kleiner werdendem Druck auch kleiner werden.

In der EP-B 295 551 wird im angegebenen Beispiel unter b) ein Formylvaleriansäureestergemisch durch fraktionierende Destillation ohne Angabe von Versuchsparametern wie Druck und Temperatur aufgetrennt. Angaben über den Gehalt an 4-Formylvaleriansäureester in der 5-FVSE-Fraktion fehlen, doch kann vermutet werden, daß er deutlich größer als 100 ppm (bezogen auf die Menge an 5-FVSE) war: bei der fraktionierten Destillation in Schritt b) des in der EP-B 295551 angegebenen Beispiels fielen über 3 Gew.-% (10 g) eines Rückstandes an. Ein so hoher Anteil an Rückstand kann nur mit der thermischen Zersetzung der Formylvaleriansäureester bei der Destillation erklärt werden. Dies wiederum läßt den Schluß zu, daß bei relativ hohen Temperaturen destilliert wurde und letztlich bei Normaldruck oder nur wenig vermindertem Druck. Unter solchen Bedingungen lassen sich die Formylvaleriansäureester wegen der geringen Siedepunktsdifferenz wie oben gezeigt wurde nur schlecht trennen. Somit enthielt die 5-FVSE-Fraktion mit an Sicherheit grenzender Wahrscheinlichkeit deutliche Anteile, d.h. größer als 100 ppm, an den isomeren Formylvaleriansäureestern, insbesondere des 4-Formylvaleriansäureesters, da dieser in der Regel in größeren Mengen als die entsprechende in 3-Stellung substituierte Verbindung vorliegt. Als weiteres Indiz für die schlechte Trennung der isomeren Verbindungen dient die Zusammensetzung der zweiten Fraktion: hier wurden 2 Gew.-% 5-, 70 Gew.-% 4- und 28 Gew.-% 3-Formylvaleriansäuremethylester erhalten.

Weitere Nachteile der in der EP-B 295 551 beschriebenen fraktionierten Destillation der Formylvaleriansäureester sind der Verlust an Wertprodukt, 5-FVSE (2 Gew.-% in der zweiten Fraktion), sowie die Bildung eines Rückstandes.

Aufgabe der vorliegenden Erfindung war daher die Bereitstellung eines Verfahrens zur effektiven und effizienten Abtrennung von 5-FVSE aus einem Gemisch mit seinen isomeren 3- und 4-Formylvaleriansäureestern in einer Reinheit von größer als 98% und einem Gehalt an 4-Formylvaleriansäureester nicht größer als 100 ppm (bezogen auf die Menge an 5-FVSE). Des weiteren sollte auch ein Verfahren zur Verfügung gestellt werden, das es gestattet, den 5-FVSE aus dem bei der Hydroformylierung von 4-Pentensäureester erhaltenen Reaktionsgemisch in der oben genannten Reinheit zu isolieren. Ferner sollte die Ausbeute an 5-FVSE nicht kleiner als 90 % betragen.

Demgemäß wurde ein verbessertes Verfahren zur Herstellung von 5-Formylvaleriansäureester in einer Ausbeute von nicht kleiner als 90 % durch Destillation eines Formylvaleriansäureestergemisches aus 5-Formylvaleriansäureester und entweder 3- oder 4-Formylvaleriansäureester oder einem Gemisch aus 3- und 4-Formylvaleriansäureestern, wobei die Esterreste der jeweiligen Formylvaleriansäureester identisch sind, gefunden, indem man die 3- oder 4-Formylvaleriansäureester oder deren Gemische bei einem Druck im Bereiche von $2 \times 10^2$ Pa bis $1 \times 10^4$ Pa (2 bis 100 mbar) und einer Temperatur von nicht über 150°C (gemessen als Kolonnensumpftemperatur) in einer Destillationskolonne vom 5-Formylvaleriansäureester abtrennt, und als Ester die entsprechenden Methyl- oder Ethylester einsetzt, und wobei die Reinheit des 5-Formylvaleriansäureesters nicht kleiner als 98 % beträgt und als Verunreinigung 4-Formylvaleriansäureester in einer Menge von nicht größer als 100 ppm vorhanden ist.

In Anbetracht dessen, daß der weiter oben genannte Stand der Technik und das allgemeinen Fachwissen eine destillative Trennung, insbesondere unter vermindertem Druck, nicht nur nicht nahelegen, sondern von einem solchen Verfahren wegzeigen, war es überraschend, daß bei der Suche nach einer Lösung des vorliegenden Problems gefunden wurde, daß bei einer Druckverminderung die Siedepunktsdifferenzen der isomeren Formylvaleriansäureester, insbesondere der $C_1$-$C_2$-Alkyl-Verbindungen, nicht kleiner, sondern größer werden.

Des weiteren wurde ein Verfahren gefunden, wobei man anstelle des Formylvaleriansäureestergemisches ein Gemisch, erhältlich aus dem Austrag einer Hydroformylierung von 4-Pentensäureester oder einem Gemisch von 2-, 3- und/oder 4-Pentensäureestern, bestehend aus

(a) einem Formylvaleriansäureestergemisch,
(b) den oder dem eingesetzten Pentensäureester(n),
(c) Valeriansäureester,
(d) Hochsiedern und
(e) Leichtsiedern

einsetzt und

(1) zunächst Leichtsieder, den oder die Pentensäureester und Valeriansäureester bei einem Druck im Bereich von 10 bis 300 mbar und Sumpftemperaturen im Bereich von nicht größer als 150°C destillativ in einer ersten Destillationskolonne (Leichtsiederkolonne) über Kopf abtrennt,

(2) den verbleibenden Sumpf einer weiteren Destillationskolonne (Isomerenkolonne) zuführt und bei einem Druck im Bereich von $2 \times 10^2$ Pa bis $1 \times 10^4$ Pa (2 bis 100 mbar) und Sumpftemperaturen im Bereich von nicht größer als 150°C über Kopf 3- und/oder 4-Formylvaleriansäureester abtrennt und

(3) den verbleibenden Sumpf einer weiteren Destillationskolonne (Reinkolonne) zuführt und bei einem Druck im Bereich von 1 bis 20 mbar und Sumpftemperaturen im Bereich von nicht größer als 150°C über Kopf 5-Formylvaleriansäureester abtrennt.

Formylvaleriansäureestergemische, bestehend aus 5-Formylvaleriansäureester und entweder 3- oder 4- Formylvaleriansäureester oder einem Gemisch aus 3- und 4-Formylvaleriansäureestern, wobei die Esterreste der jeweiligen Formylvaleriansäureester identisch sind, erhält man in der Regel durch katalytische Hydroformylierung des entsprechenden 4-Pentensäureesters oder des entsprechenden 3-Pentensäureesters, der in einem ersten Reaktionsschritt zu 2- und 4-Pentensäureester isomerisiert wird, so daß ein Gemisch aus 2-, 3- und 4-Pentensäureester vorliegt, und wobei in einem nachfolgenden Reaktionsschritt der 4-Pentensäureester mit hoher Regioselektivität endständig hydroformyliert wird.

Alternativ kann die Isomerisierung auch als ein separater, der Hydroformylierung vorgelagerter Verfahrensschritt ausgeführt werden, wobei der im Gleichgewicht nur in geringer Konzentration vorliegende 4-Pentenester destillativ zumindest angereichert werden muß. Die Hydroformylierung von Pentensäureestern zu 5-Formylvaleriansäureestern erfordert im allgemeinen als Katalysator eine Metallverbindung der VIII. Nebengruppe, die unter Synthesebedingungen zur Bildung von Metallcarbonylkomplexen befähigt ist. Bevorzugt werden Cobalt- oder Rhodiumverbindungen, die durch Liganden wie Phosphine oder Phosphite modifiziert sein können. Abhängig von der Zusammensetzung der Pentenesterisomeren haben sich folgende, bevorzugte Verfahren bewährt:

1. Cobaltkatalysatoren

Cobaltverbindungen, d.h. Cobaltcarbonyle oder Vorstufen, die unter Reaktionsbedingungen in Cobaltcarbonyle überführt werden können, vermögen üblicherweise Pentenester, auch Isomerenmischungen mit 2- und 3-Pentenestern (PSE), bei Umsätzen < 70 % mit rund 95 % Selektivität in Formylvalerianester zu überführen, wobei der n-Anteil gemäß der EP-B 295 554 bis zu 70 % betragen kann. Höhere Umsätze sind ebenfalls möglich, führen aber nach bisherigen Beobachtungen wegen verstärkter Nebenproduktbildung zu Selektivitätsverlusten.

2. Rhodium/Triphenylphosphinkatalysatoren

Mit diesen Katalysatoren kann gemäß der EP-B 125 567 von den Pentenesterisomeren ausschließlich 4-Pentensäureester in 5-Formylvaleriansäureester überführt werden. Bei Oxierung von Pentenestergemischen reagierten entsprechend nur die 4-Pentenester, es werden folgende Schritte ausgeführt:

a) die Isomerisierung des 3-PSE zu Mischungen isomerer PSE und die destillative Anreicherung von 4-PSE auf etwa 95 % (detailliert in der EP-B 125 567 beschrieben),

b) die selektive Hydroformylierung des 4-PSE zu überwiegend 5-FVSE (eingesetzt wird der Hydroformylierungs-katalysator Rhodium/P(C$_6$H$_5$)$_3$), und die

c) destillative Abtrennung der Formylvalerianester und Rückführung der PSE in Schritt a).

3. Die WO 94/26688 beschreibt wasserlösliche Rhodium/Phosphinkatalysatoren

Mit diesen Katalysatoren kann ebenfalls von den Pentenesterisomeren ausschließlich 4-Pentensäureester in 5-Formylvaleriansäureester überführt werden. Bei Oxierung von Pentenestergemischen reagiert entsprechend nur 4-Pentenester. Das Verfahren besteht aus den gleichen drei Schritten wie unter 2. genannt:

a) Isomerisierung des 3-PSE zu Mischungen isomerer PSE; destillative Anreicherung von 4-PSE,

b) Selektive Hydroformylierung des 4-PSE zu überwiegend 5-FVSE; (bevorzugt wird der wasserlösliche Hydro-formylierungskatalysator Rhodium/P(m-C$_6$H$_4$SO$_3$Na)$_3$),

c) destillative Abtrennung der Formylvalerianester und Rückführung der PSE in Schritt a).

4. Die EP-A 556 681 beschreibt Rhodium/Chelatphosphitkatalysatoren

Besonders vorteilhaft gelingt die Hydroformylierung von Pentenestern zu 5-Formylvalerianestern mittels Rh/ Chelatphosphitkatalysatoren. Werden interne Pentenester eingesetzt, erfolgt üblicherweise im gleichen Verfahrensschritt vor der eigentlichen Hydroformylierung die Isomerisierung der Pentenester. Wegen der im Vergleich zu den übrigen Isomeren wesentlich höheren Reaktivität des 4-PSE, wird dieser bei weitem bevorzugt hydroformyliert, so daß man selektiv 5-FVSE erhält. In Beispielen belegt wird der Einsatz von 4-, 3- und 2-PSE sowie eine Mischung aus 3- und 4-PSE. Es wird eine Regioselektivität zu 5-Formylvalerianester von bis zu 94 % (Einsatz cis/trans-3-Pentenester) erhalten.

Geeignete Pentensäureester leiten sich von Alkanolen mit 1 bis 12 Kohlenstoffatomen oder Cycloalkanolen mit 5 bis 8 Kohlenstoffatomen ab. Besonders bevorzugt sind Pentensäure-C$_1$-C$_{12}$-alkylester, insbesondere Penten-säure-C$_1$-C$_4$-alkylester, z.B. Pentensäuremethylester. Geeignete Verbindungen sind z.B. 4-Pentensäureester, 3-Pentensäureester und 2-Pentensäureester einzeln oder Gemische derselben. Beispielsweise seien genannt Methyl-, Ethyl-, Propyl-, Isopropyl-, Butyl-, Hexyl-, Nonyl-, Dodecyl-, Cyclopentyl- oder Cyclohexylester der 2-, 3- oder 4-Pentensäure, besonders bevorzugt die Methyl- und Ethylester.

Erfindungsgemäß destilliert man ein Formylvaleriansäureestergemisch aus 5-Formylvaleriansäureester und entweder 3- oder 4-Formylvaleriansäureester oder, bevorzugt, ein Gemisch aus 3- und 4-Formylvaleriansäureestern, wobei die Esterreste der jeweiligen Formylvaleriansäureester identisch sind, und trennt dabei die 3- oder 4-Formylvaleriansäureester oder deren Gemische bei einem Druck im Bereich von 2x10$^2$ bis 10$^4$ (2 bis 100), insbesondere von 5x10$^2$ bis 5x10$^3$ Pa (5 bis 50 mbar) und einer Temperatur von nicht über 150°C, bevorzugt im Bereich von 100 bis 130°C (gemessen als Kolonnensumpftemperatur) in einer Destillationskolonne vom 5-Formylvaleriansäureester ab. Erfindungsgemäß setzt man Methyl- oder Ethylester ein.

Ein bevorzugtes einzusetzendes Gemisch hat folgende Zusammensetzung:

von 60 bis 98, insbesondere von 80 bis 96 Gew.-% 5-Formylvaleriansäuremethylester,

von 1 bis 20, insbesondere von 2 bis 10 Gew.-% 4-Formylvaleriansäuremethylester,

von 1 bis 20, insbesondere von 2 bis 10 Gew.-% 3-Formylvaleriansäuremethylester und

von 0 bis 2, insbesondere von 0 bis 1 Gew.-% Hochsieder.

Der im Sumpf zurückbleibende 5-FVSE weist erfindungsgemäß eine Reinheit von nicht kleiner als 98%, bevorzugt von nicht kleiner als 98,5% auf, und besitzt den 4-Formylvaleriansäureester in einer Menge von kleiner als 100, insbesondere kleiner als 80 ppm als Verunreinigung.

Als Destillationsvorrichtung verwendet man üblicherweise eine Destillationskolonne, bevorzugt eine Pak-kungskolonne mit einer theoretischen Bodenzahl von mindestens 30, insbesondere zwischen 35 und 50. In einer besonders bevorzugten Ausführungsform verwendet man eine Packungskolonne, in der das Packungsmaterial eine geordnete Struktur aufweist. Solche Packungsmaterialien sind im Handel erhältlich, beispielsweise von der Fa. Sulzer mit der Bezeichnung DX oder DY.

In einer besonders bevorzugten Ausführungsform setzt man anstelle des Formylvaleriansäureestergemisches ein Gemisch, erhältlich aus dem Austrag einer Hydroformylierung von 4-Pentensäureester oder einem Gemisch von isomeren 2-, 3- und/oder 4-Pentensäureestern, bestehend aus

(a) einem Formylvaleriansäureestergemisch,
(b) den oder dem eingesetzten Pentensäureester(n),
(c) Valeriansäureester,
(d) Hochsiedern und
(e) Leichtsiedern

ein und

(1) trennt zunächst Leichtsieder, den oder die Pentensäureester und Valeriansäureester bei einem Druck im Bereich von $1 \times 10^3$ bis $3 \times 10^4$ Pa (10 bis 300), bevorzugt $2 \times 10^3$ bis $1 \times 10^4$ Pa (20 bis 100 mbar) (gemessen als Kolonnenkopf-druck) und Sumpftemperaturen im Bereich von nicht größer als $150°C$, insbesondere im Bereich von 100 bis $130°C$, destillativ in einer ersten Destillationskolonne (Leichtsiederkolonne) über Kopf ab,
(2) führt dann den verbleibenden Sumpf einer weiteren Destillationskolonne (Isomerenkolonne) zu und trennt bei einem Druck im Bereich von $2 \times 10^2$ bis $1 \times 10^4$ (2 bis 100), insbesondere von $5 \times 10^2$ bis $5 \times 10^3$ Pa (5 bis 50 mbar) (gemessen als Kolonnenkopfdruck) und Sumpftemperaturen im Bereich von nicht größer als $150°C$, insbesondere im Bereich von 100 bis $130°C$ über Kopf 3- und/oder 4- Formylvaleriansäureester ab, und
(3) führt anschließend den verbleibenden Sumpf einer weiteren Destillationskolonne (Reinkolonne) zu und trennt bei einem Druck im Bereich von $1 \times 10^2$ bis $2 \times 10^3$ (1 bis 20), insbesondere von $1 \times 10^2$ bis $1 \times 10^3$ Pa (1 bis 10 mbar) (gemessen als Kolonnenkopfdruck) und Sumpftemperaturen im Bereich von nicht größer als $150°C$, insbesondere im Bereich von 100 bis $130°C$, über Kopf 5-Formylvaleriansäureester ab.

Als Leichtsieder werden alle "leichter" als die Formylvaleriansäureester siedenden Verbindungen bezeichnet, d.h. solche mit einem niedrigeren Siedepunkt. Entsprechend umfaßt der Begriff Hochsieder alle diejenigen Verbindungen, die einen höheren Siedepunkt als 5-FVSE aufweisen.
Nach dem erfindungsgemäß bevorzugten Verfahren erhält man 5-FVSE in einer Reinheit von mindestens 98%, bevorzugt größer als 98,5 % und einer Menge an 4-Formylvaleriansäureester als Verunreinigung von nicht größer als 100, insbesondere nicht größer als 80 ppm, bezogen auf die Menge an 5-FVSE.
Im Falle, daß das Formylvaleriansäureestergemisch mittels homogener Rhodium-Katalyse hergestellt wird, und sich der Katalysator somit nach der Hydroformylierung noch im Reaktionsgemisch befindet, empfiehlt sich eine Abtrennung des Katalysators vor der oben genannten ersten Destillationsstufe, indem man bevorzugt den Hydroformylierungsaustrag bei einer Temperatur von bevorzugt nicht größer als $120°C$ und einem Druck von-im allgemeinen nicht größer als $3 \times 20^3$ Pa (30 mbar) destilliert, und dann wie oben beschrieben weiterverfährt.
Eine weitere bevorzugte Ausführungsform betrifft die Ausführung der oben beschriebenen Verfahrensschritte in Gegenwart von möglichst wenig Sauerstoff, bevorzugt unter Sauerstoffausschluß.
Hierzu kann man Destillationsvorrichtungen einsetzen, die an den Kontaktstellen, an denen die einzelnen miteinander zu verbindenden Teile zusammenstoßen, verschweißt sind. Desgleichen kann man bei Verwendung von Flanschverbindungen ein Eindringen von Luftsauerstoff von außen in die Vorrichtung durch Anlegen von entsprechenden Schutzgashüllen um die Flansche verhindern oder minimieren.
Für die praktische Durchführung kann ein gewisser Sauerstoffanteil akzeptiert werden, wobei sich in der Regel der $O_2$-Gehalt/Stunde nach dem gewünschten Zersetzungsgrad von 5-FVSE und der Dauer der Destillation richtet. Als Anhaltspunkt kann die empirisch gefundene Gleichung I

$$\% \text{ Zersetzung 5-FVSE} = (3,96 \cdot 10^{-4} \cdot a + 1,8) \cdot t \qquad\qquad \text{I}$$

wobei

$a = O_2$-Gehalt in ppm/h und
$t$ = Zeit in h

bedeuten, für $130°C$ und $0 < t < 5$ h
herangezogen werden. Sollte z.B. die Zersetzung von 5-FVSE nicht größer als 5 % während 2 1/2 h betragen, so könnte ein $O_2$-Gehalt von nicht größer als 505 ppm/h zugelassen werden.

Das erfindungsgemäße Verfahren hat den Vorteil gegenüber Verfahren aus dem Stand der Technik, daß 5-FVSE großtechnisch in hoher Reinheit und mit einem für die spätere Weiterverarbeitung, insbesondere zur Herstellung von Polycaprolactamfäden, vernachlässigbaren Gehalt an 4-Formylvaleriansäureester zur Verfügung gestellt werden kann.

Beispiele

Bei sämtlichen Beispielen bezieht sich der Ausdruck "Ester" immer auf den Methylester.

Beispiel 1

120 kg eines Hydroformylierungsaustrags, dessen Zusammensetzung in der untenstehenden Tabelle wiedergegeben ist, wurde destillativ aufgearbeitet. Die Leichtsieder wurden in einer Pakkungskolonne (Sulzer CY, 10 theoret. Böden) bei einem Kolonnenkopfdruck von $4 \times 10^3$ Pa(40 mbar) und einer Sumpftemperatur von 113°C abgetrennt, die Isomerentrennung erfolgte in einer Packungskolonne (Sulzer DX, 40 theoret. Böden) bei einem Kolonnenkopfdruck von 12 mbar und einer Sumpftemperatur von 112,5°C, die gebildeten Hochsieder wurden in einem Dünnschichtverdampfer mit aufgesetzter packungskolonne (Sulzer CY, 20 theoret. Böden) bei einem Kolonnenkopfdruck von 4 mbar und einer Sumpftemperatur von 130°C als Sumpfprodukt vom 5-FVSE abgetrennt. Es resultierte eine 5-FVSE-Gesamtausbeute von 94 % (67,7 kg) bezogen auf die 5-FVSE-Menge im Zulauf zur Aufarbeitung. Die 5-FVSE-Reinheit betrug 98,5 %. Der 4-FVSE-Gehalt lag bei 80 ppm (bez. auf 5-FVSE). Die Ausbeute an 3-/4-FVSE Isomerengemisch betrug 99 % (11,8 kg).

Die Zersetzung von 5-FVSE durch Sauerstoff war im durchgeführten Versuch nicht größer als 6 % (Gesamtausbeute: 94 %). Nach der empirisch ermittelten Gleichung I ergibt sich für eine Destillationsdauer von t = 3 h eine maximale $O_2$-Konzentration von 510 ppm $O_2$/h.

Beispiel 2

Beispiel 1 wurde wiederholt, jedoch wurde der Kolonnenkopfdruck in der Isomerentrennkolonne auf 20 mbar erhöht, wobei die Sumpftemperatur 132°C betrug. Die 5-FVSE-Gesamtausbeute sank auf 92 % der zugeführten 5-FVSE-Menge. Die Reinheit des 5-FVSE betrug 98,5 %, der Gehalt an 4-FVSE 80 ppm (bez. auf 5-FVSE).

Vergleichsbeispiel 1

Beispiel 1 wurde wiederholt, jedoch wurde der Kolonnenkopfdruck der Hochsiederabtrennung auf $8 \times 10^2$ Pa (8 mbar) erhöht. Die erforderliche Sumpftemperatur betrug 158°C. Die 5-FVSE-Gesamtausbeute sank auf 89 % der zugeführten 5-FVSE-Menge.

Vergleichsbeispiel 2

100 g 5-FVSE wurden mit 7 mg Sauerstoff pro g FVSE und Stunde bei einer Temperatur von 130°C über einen Zeitraum von 3 h erhitzt. Es zersetzten sich dabei 20 Gew.-% des eingesetzten 5-FVSE.

Vergleichsbeispiel 2 wurde bei unterschiedlichen Verweilzeiten und einem Sauerstoffgehalt von kleiner als 10 ppm (bezogen auf 5-FVSE) wiederholt:

nach 1 h Verweilzeit waren 1 Gew.-% des 5-FVSE zersetzt,
nach 3 h Verweilzeit waren 4 Gew.-% des 5-FVSE zersetzt, und
nach 7 h Verweilzeit waren 8,5 Gew.-% des 5-FVSE zersetzt.

Herstellung des Hydroformylierungsaustrags

637 kg eines Gemisches aus
0,2 Gew.-% 2-cis-Pentensäuremethylester,
71, 5 Gew.-% 3-cis-Pentensäuremethylester,
23,8 Gew.-% 3-trans-Pentensäuremethylester,
0,85 Gew.-% 2-trans-Pentensäuremethylester, und
3 Gew.-% β-Picolin sowie ca. 0,6 Gew.-% nicht identifizierte Verunreinigungen wurden in Gegenwart eines handelsüblichen Rhodiumkatalysators (Rh-Gehalt: 120 ppm, bezogen auf das Gemisch) bei einem Druck von 4 bar und einer Temperatur von 100°C in einer Zweikesselkaskade bei einer Verweilzeit von 5 h einer Synthesegasatmosphäre (CO/H$_2$-Vol.-Verhältnis 1:1) ausgesetzt. Nach dem Abkühlen und Entspannen auf Umgebungsdruck wurde der erhaltene Hydroformylierungsaustrag in den entsprechenden Beispielen eingesetzt. Die Zusammensetzung des Austrags ergibt sich aus untenstehender Tabelle.

Tabelle:

| Zusammensetzung des Hydroformylierungsaustrags | |
|---|---|
| Verbindung | Gehalt im Zulauf (in Gew.-%) |
| Leichtsiederanteil | 2,96 |
| PSE | 26,83 |
| 3-FVSE | 4,66 |
| 4-FVSE | 5,27 |
| 5-FVSE | 60,04 |
| Hochsiederanteil | 0,22 |

**Patentansprüche**

1. Verfahren zur Herstellung von 5-Formylvaleriansäureestern in einer Ausbeute von nicht kleiner als 90 % durch Destillation eines Formylvaleriansäureestergemisches aus 5-Formylvaleriansäureester und entweder 3- oder 4-Formylvaleriansäureester oder einem Gemisch aus 3- und 4-Formylvaleriansäureestern, wobei die Esterreste der jeweiligen Formylvaleriansäureester identisch sind, dadurch gekennzeichnet, daß man die 3- oder 4-Formylvaleriansäureester oder deren Gemische bei einem Druck im Bereich von 2x10$^2$ bis 1x10$^4$ Pa (2 bis 100 mbar) und einer Temperatur von nicht über 150°C (gemessen als Kolonnensumpftemperatur) in einer Destillationskolonne vom 5-Formylvaleriansäureester abtrennt, und als Ester die entsprechenden Methyl- oder Ethylester einsetzt, und wobei die Reinheit des 5-Formylvaleriansäureesters nicht kleiner als 98 % beträgt und als Verunreinigung 4-Formylvaleriansäureester in einer Menge von nicht größer als 100 ppm vorhanden ist.

2. Verfahren gemäß Anspruch 1, dadurch gekennzeichnet, daß man anstelle des Formylvaleriansäureestergemisches ein Gemisch, erhältlich aus dem Austrag einer Hydroformylierung von 4-Pentensäureester oder einem Gemisch von 2-, 3- und/oder 4-Pentensäureestern, bestehend aus

    (a) einem Formylvaleriansäureestergemisch,
    (b) den oder dem eingesetzten Pentensäureester(n),
    (c) Valeriansäureester,
    (d) Hochsiedern und
    (e) Leichtsiedern

einsetzt und

    (1) zunächst Leichtsieder, den oder die Pentensäureester und Valeriansäureester bei einem Druck im Bereich von 10 bis 300 mbar und Sumpftemperaturen im Bereich von nicht größer als 150°C destillativ in einer ersten Destillationskolonne (Leichtsiederkolonne) über Kopf abtrennt,
    (2) den verbleibenden Sumpf einer weiteren Destillationskolonne (Isomerenkolonne) zuführt und bei einem Druck im Bereich von 2 bis 100 mbar und Sumpftemperaturen im Bereich von nicht größer als 150°C über Kopf 3- und/oder 4-Formylvaleriansäureester abtrennt und
    (3) den verbleibenden Sumpf einer weiteren Destillationskolonne (Reinkolonne) zuführt und bei einem Druck im Bereich von 1x10$^2$ bis 2x10$^3$ Pa (1 bis 20 mbar) und Sumpftemperaturen im Bereich von nicht größer als 150°C über Kopf 5-Formylvaleriansäureester abtrennt.

3. Verfahren nach den Ansprüchen 1 oder 2, dadurch gekennzeichnet, daß man die Verfahrensschritte in Gegenwart von möglichst wenig Sauerstoff, bevorzugt unter Sauerstoffausschluß, durchführt.

**EP 0 850 213 B1**

**Claims**

1. A process for preparing 5-formylvaleric esters in a yield of not less than 90% by distillation of a formylvaleric ester mixture of 5-formylvaleric ester and either 3- or 4-formylvaleric ester or a mixture of 3- and 4-formylvaleric esters, where the ester radicals of the respective formylvaleric esters are identical, which comprises separating the 3- or 4-formylvaleric ester or a mixture thereof from the 5-formylvaleric ester in a distillation column at a pressure in the range from $2 \times 10^2$ to $1 \times 10^4$ Pa (2 to 100 mbar) and a temperature of not above 150°C (measured as the temperature at the bottom of the column) and using as esters the corresponding methyl or ethyl esters, where the purity of the 5-formylvaleric ester is not less than 98% and, as impurity, 4-formylvaleric ester is present in an amount of not more than 100 ppm.

2. A process as claimed in claim 1, wherein the formylvaleric ester mixture used is replaced by a mixture obtainable from the output of a hydroformylation of 4-pentenoic ester or a mixture of 2-, 3- and/or 4-pentenoic esters, comprising

   (a) a formylvaleric ester mixture,
   (b) the pentenoic ester(s) used,
   (c) valeric ester,
   (d) high boilers and
   (e) low boilers

   and

   (1) low boilers, the pentenoic ester(s) and valeric ester are first separated off by distillation via the top in a first distillation column (low boiler column) at a pressure in the range from 10 to 300 mbar and bottom temperatures in the range of not greater than 150°C,
   (2) the remaining bottoms are fed to a further distillation column (isomer column) and 3- and/or 4-formylvaleric esters are separated off via the top at a pressure in the range from 2 to 100 mbar and bottom temperatures in the range of not greater than 150°C and
   (3) the remaining bottoms are fed to a further distillation column (pure column) and 5-formylvaleric ester is separated off via the top at a pressure in the range from $1 \times 10^2$ to $2 \times 10^3$ Pa (1 to 20 mbar) and bottom temperatures in the range of not greater than 150°C.

3. A process as claimed in claim 1 or 2, wherein the process steps are carried out in the presence of very little oxygen, preferably with exclusion of oxygen.

**Revendications**

1. Procédé de préparation d'esters d'acide 5-formylvalérique en un rendement qui n'est pas inférieur à 90%, par distillation d'un mélange d'esters d'acide formylvalérique à base d'ester d'acide 5-formylvalérique et d'ester d'acide soit 3-, soit 4-formylvalérique ou d'un mélange d'esters d'acides 3- et 4-formylvalériques, les radicaux ester des esters d'acide formylvalérique respectifs étant identiques, caractérisé en que, dans une colonne de distillation, on isole, de l'ester d'acide 5-formylvalérique, les esters d'acide 3- ou 4-formylvalérique ou leurs mélanges à une pression de l'ordre de $2 \times 10^2$ à $1 \times 10^4$ Pa (2 à 100 mbars) et à une température qui n'est pas supérieure à 150°C (mesurée sous la forme de température de fond de colonne) et on met en oeuvre, comme esters, les esters méthyliques ou éthyliques correspondants, la pureté de l'ester d'acide 5-formylvalérique n'étant pas inférieure à 98% et l'ester d'acide 4-formylvalérique étant présent comme impureté en une quantité qui n'est pas supérieure à 100 ppm.

2. Procédé suivant la revendication 1, caractérisé en ce que, au lieu du mélange d'esters d'acide formylvalérique, on met en oeuvre un mélange, que l'on peut obtenir de la sortie d'une hydroformylation d'ester d'acide 4-penténoïque ou d'un mélange d'esters d'acides 2-, 3- et/ou 4-penténoïques, et qui est constitué

   (a) d'un mélange d'esters d'acide formylvalérique,
   (b) du ou des esters d'acide penténoïque mis en oeuvre,
   (c) d'ester d'acide valérique,
   (d) d'agents à point d'ébullition élevé, et

8

(e) d'agents à bas point d'ébullition,

et

(1) en ce qu'on isole tout d'abord par distillation dans une première colonne de distillation (colonne des agents à bas point d'ébullition), par la tête, des agents à bas point d'ébullition, le ou les esters d'acide penténoïque et esters d'acide valérique, à une pression de l'ordre de 10 à 300 mbars et à des températures de fond de colonne d'un ordre qui n'est pas supérieur à 150°C,

(2) on amène le fond de colonne subsistant à une autre colonne de distillation (colonne d'isomères) et on isole par la tête des esters d'acides 3- et/ou 4-formylvalériques à une pression de l'ordre de 2 à 100 mbars et à des températures de fond de colonne d'un ordre qui n'est pas supérieur à 150°C, et

(3) on amène le fond de colonne subsistant à une autre colonne de distillation (colonne de produit pur) et on isole par la tête de l'ester d'acide 5-formylvalérique à une pression de l'ordre de $1 \times 10^2$ à $2 \times 10^3$ Pa (1 à 20 mbars) et à des températures de fond de colonne d'un ordre qui n'est pas supérieur à 150°C.

3. Procédé suivant l'une des revendications 1 et 2, caractérisé en ce qu'on effectue les étapes du procédé en présence d'aussi peu que possible d'oxygène, de préférence à l'exclusion d'oxygène.